(19) Europäisches Patentamt
European Patent Office
Office européen des brevets

(11) Publication number: **0 048 024**
**A1**

# (12) EUROPEAN PATENT APPLICATION

(21) Application number: 81107301.4

(22) Date of filing: 16.09.81

(51) Int. Cl.$^3$: C 07 D 401/12

(30) Priority: 16.09.80 US 187719

(43) Date of publication of application: 24.03.82 Bulletin 82/12

(84) Designated Contracting States: BE DE FR GB IT NL

(71) Applicant: E.I. DU PONT DE NEMOURS AND COMPANY
Legal Department 1007 Market Street
Wilmington Delaware 19898(US)

(72) Inventor: Ittel, Steven Dale
2504 Raven Road Chalfonte II
Wilmington Delaware 19810(US)

(72) Inventor: Peet, William George
118 Sharpless Drive
Newark Delaware 19711(US)

(74) Representative: Abitz, Walter, Dr.-Ing. et al,
Abitz, Morf, Gritschneder P.O. Box 86 01 09
D-8000 München 86(DE)

## (54) Improved process for producing 1,3-bis(2-pyridylimino)isoindolines and compositions containing transition metal complexes thereof.

(57) There are disclosed an improved process for preparing a composition consisting essentially of at least one transition metal complex of a 1,3-bis(2-pyridylimino)isoindoline comprising contacting in a molten phase a 1,2-dicyanobenzene with at least one 2-aminopyridine in the presence of a transition metal complex and an improved process for preparing the 1,3-bis(2-pyridylimino)isoindoline ligand from the same starting materials comprising contacting in a molten phase said starting materials, optionally in the presence of an alkaline earth metal carbonate.

EP 0 048 024 A1

TITLE

# IMPROVED PROCESS FOR PRODUCING 1,3-BIS(2-PYRIDYLIMINO)ISOINDOLINES AND COMPOSITIONS CONTAINING TRANSITION METAL COMPLEXES THEREOF

## BACKGROUND OF THE INVENTION

### Field of the Invention

This invention relates to an improved process for preparing a composition containing a 1,3-bis(2-pyridylimino)isoindoline transition metal complex and an improved process for the preparation of the isoindoline ligand themselves.

More specifically, this invention is related to the direct production of these transition metal complexes from 1,2-dicyanobenzene and 2-aminopyridines and to the production of the ligand themselves from the same starting materials.

### Description of the Prior Art

Siegl, J. Org. Chem. 42, 1872 (1977), discloses the preparation of 1,3-bis(arylimino)isoindolines, in specific the 1,3-bis(2-pyridylimino) compounds, by reacting a primary aromatic amine with phthalonitrile in alcoholic solvent using as a catalyst an alkaline earth metal salt, such as $CaCl_2$, $Mg(ClO_4)_2$ or $MgI_2$. Reaction times of 24-48 hours are given. The reference also discloses that metal chelate complexes, MLX, can be prepared directly by using certain divalent transition metal acetates and chlorides to facilitate the addition of primary aromatic amines to phthalonitrile. Specifically mentioned are complexes such as the 1,3-bis(2-pyridylimino)isoindoline complexes where (1) M is Co, Ni or Zn and X is acetate; (2) M is Ni and X is chloride; and (3) the Cu complex of 1,3-bis(4-methylpyridylimino)-isoindoline where X is acetate. Reaction times of 6-48 hours were given.

[CR 8010]

Elvidge and Linstead, J. Chem. Soc., 5000 and 5008 (1952), disclose the preparation of arylimino isoindolines including 1,3-bis(2-pyridylimino)isoindoline from diiminoisoindoline and 2-aminopyridine. Certain metal complexes are also disclosed.

Robinson et al, Inorg. Chem., 6, 392 (1967), disclose the preparation of 1,3-bis(2-pyridylimino)-isoindoline by refluxing 2-aminopyridine and 1,3-diiminoisoindoline in butanol for 4 hours. The reference also discloses the preparation of transition metal complexes of this ligand by reacting the preformed ligand with the transition metal perchlorate, chloride or acetate.

Gagne et al, Inorg. Chem., 18, 771 (1979), disclose the preparation of 1,3-bis[2-(4-methylpyridyl)-imino]isoindoline, the complex of this ligand with copper acetate and a Cu(I) carbonyl complex of the ligand.

Prior art processes for the preparation of the 1,3-bis(2-pyridylimino)isoindoline compounds require, in general, somewhat long reaction times, present difficult solvent separation problems and/or produce a product having a halide as an impurity. The presence of a halide is undesirable if the product is to be used to prepare a catalyst for reactions which are run in stainless-steel equipment. A process which avoids any or all of these drawbacks would fulfill a definite commercial need.

Moreover, for the preparation of transition metal complexes of 1,3-bis(2-pyridylimino)isoindolines the prior art processes involve the reaction of a suitable metal salt with a preformed ligand. A direct synthesis preparation would offer a more efficient and possibly attractive commercial process.

## SUMMARY OF THE INVENTION

The present invention provides an improved process for preparing a composition consisting essentially of at least one transition metal complex of a 1,3-bis(2-pyridylimino)isoindoline of a particular formula specified herein, comprising contacting in a molten phase a 1,2-dicyanobenzene with at least one 2-aminopyridine in the presence of a transition metal, M, carbonate or carboxylate, wherein M is Co, Mn, Fe, Ni, Pd, Zn, Cu and Ru, optionally in the presence of an alkaline earth metal carbonate, at a temperature of from about 100°-300°C. The invention further provides an improved process for preparing the 1,3-bis(2-pyridylimino)isoindoline ligand of the formula specified herein comprising contacting in a molten phase a 1,2-dicyanobenzene with at least one 2-aminopyridine, optionally in the presence of an alkaline earth metal carbonate, at a temperature of from about 150°-300°C.

## DETAILED DESCRIPTION OF THE INVENTION

The process of the invention offers a direct route to compositions containing transition metal complexes of a 1,3-bis(2-pyridylimino)isoindoline. The transition metal complexes prepared by the present process have the formula

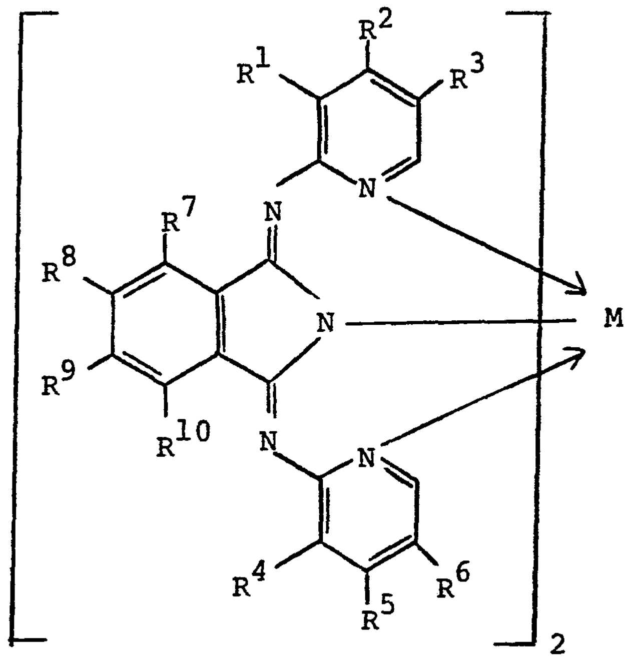

or

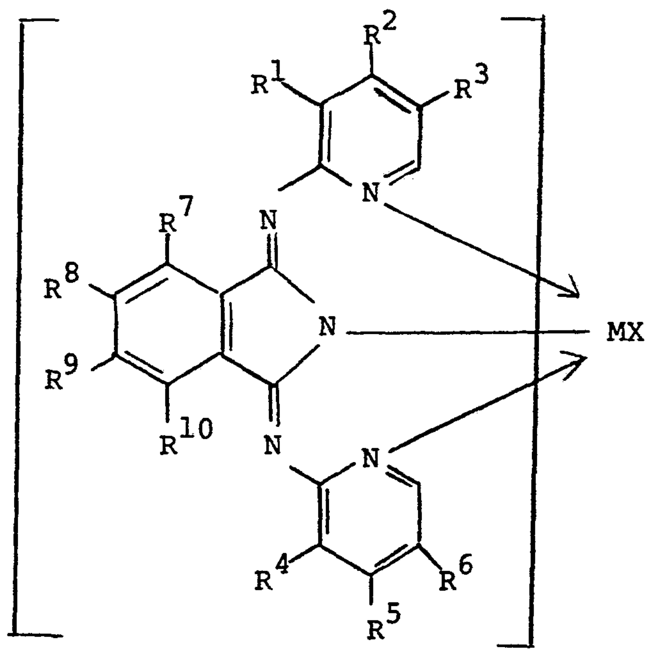

wherein

M is a divalent transition metal selected from Co, Mn, Fe, Ni, Pd, Zn, Cu and Ru;

X is a carboxylate group, such as acetate,

propionate, decanoate, adipate, 2-ethylhexanoate(octoate), naphthenate, octanoate, or 4-cyclohexylbutyrate;

$R^1$, $R^2$, $R^3$, $R^4$, $R^5$, and $R^6$ are independently hydrogen, lower alkyl, lower alkoxyalkyl, phenyl, benzyl, or phenethyl or any two adjacent members of $R^1$ through $R^6$ are jointly four CH entities of a benzene ring fused to the pyridine ring;

$R^7$, $R^8$, $R^9$, and $R^{10}$ are independently hydrogen, lower alkyl or lower alkoxyalkyl or any two adjacent members of $R^7$ through $R^{10}$ can jointly be four CH entities of a benzene ring fused to the benzene ring of the isoindoline moiety;

with the proviso that the values of $R^1$ through $R^{10}$ can be different for each ligand.

The transition metal complexes produced by the present process can alternatively be represented by formulae A or B:

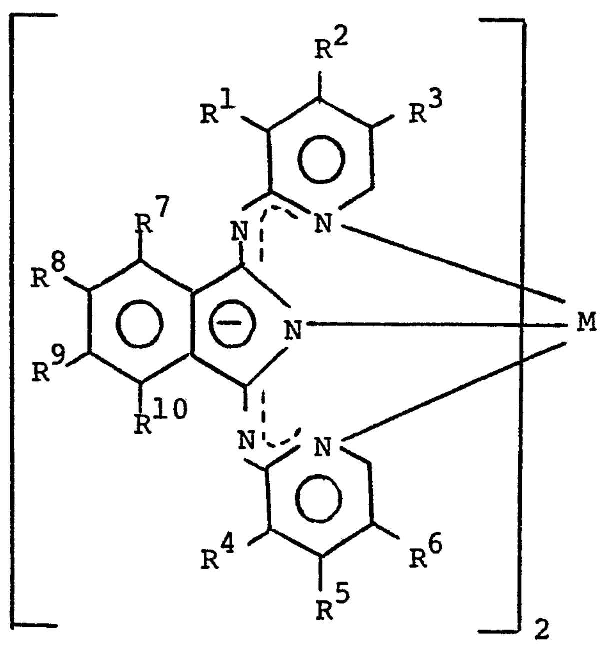

A

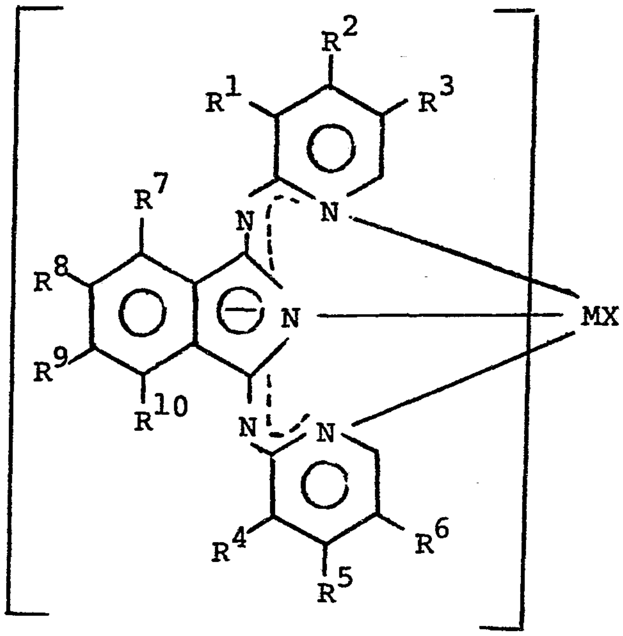

B

wherein $R^1$ through $R^{10}$, and M are as previously defined.

As used herein "lower alkyl" means alkyl of 1-6 carbon atoms and "lower alkoxyalkyl" can have from 2-6 carbon atoms. The preferred values of $R^1$ through $R^6$ are hydrogen, methyl and t-butyl. Due to availability of starting materials, preferably at least one of $R^1$, $R^2$ and $R^3$ and at least one of $R^4$, $R^5$ and $R^6$ are hydrogen; and more preferably at least two of each set of these R groups are hydrogen. Most preferably at least one of each set of these R groups is methyl or $R^2$ and $R^5$ are t-butyl and the other of these R groups are hydrogen. Again due to availability of starting materials, $R^7$ through $R^{10}$ are preferably hydrogen, methyl or t-butyl. Preferably M is Co, Mn or Fe and most preferably M is Co. X is preferably a carboxylate having up to 20 carbon atoms and most preferably is acetate, octoate or naphthenate because of industrial availability.

Examples of compositions produced by the process of the invention are the cobalt, nickel, iron,

and ruthenium derivatives of 1,3-bis(2-pyridylimino)-isoindoline, the cobalt derivative being formulated here in abbreviated form as $Co(BPI)_2$; and the cobalt, nickel, iron, ruthenium derivatives of 1,3-bis(3-methyl-2-pyridylimino)isoindoline, 1,3-bis(3,4-dimethyl-2-pyridylimino)isoindoline, 1,3-bis(3-ethyl-2-pyridylimino)isoindoline, 1,3-bis(3-butyl-2-pyridylimino)-isoindoline, 1,3-bis(4-pentyl-2-pyridylimino)isoindoline, 1,3-bis(4-isohexyl-2-pyridylimino)isoindoline, 1,3-bis(5-phenyl-2-pyridylimino)isoindoline, 5-t-butyl-1,3-bis(2-pyridylimino)isoindoline, 4,7-dimethyl-1,3-bis(3-methyl-2-pyridylimino)isoindoline, 4,5,6-trimethyl-1,3-bis(2-pyridylimino)isoindoline, 4,5,6,7-tetrakis(t-pentyl)-1,3-bis(2-pyridylimino)isoindoline, 4-hexyl-1,3-bis(2-pyridylimino)isoindoline, and 1,3-bis(1-isoquinolylimino)isoindoline. The most preferred compositions, because of availability of starting materials and catalytic activity, are $Co(BPI)_2$, the cobalt derivative of 1,2-bis(3-methyl-2-pyridylimino)isoindoline $[Co(3MeBPI)_2]$, and a mixture of cobalt(II) derivatives of bis(monomethyl-2-pyridylimino)isoindolines made from a mixture of 2-amino-3-, 4-, and 5-methylpyridines as one of the starting materials. The composition can be single complexes, mixture of isomers, or a mixture of complexes and does not need to be separated from reaction by-products in order to be used as a catalyst.

The process for preparing these transition metal complexes comprises contacting in the molten phase a 1,2-dicyanobenzene of the formula

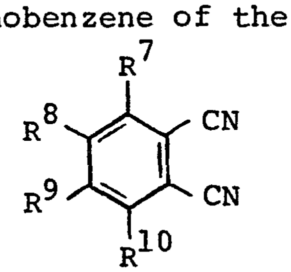 ,

where $R^7$ through $R^{10}$ are as previously defined, with at least one 2-aminopyridine of the formula

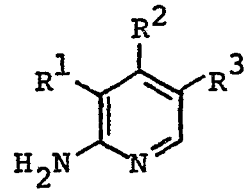
,

wherein $R^1$ through $R^3$ are as previously defined, in the presence of a transition metal, M, carbonate or a carboxylate of the formula $MX_2$ where M and X are as previously defined, and optionally in the presence of an alkaline earth metal carbonate at a temperature of from about 100°-300°C.

Preferably, in the present process for making the complexes the temperature is from about 150°C to about 220°C. The optimum temperature will vary with the metal involved in that copper, zinc, palladium and iron usually react at lower temperatures than nickel, cobalt and manganese. The ratio of the reactants is not critical and will usually be about 1:1:2 for the molar ratio of transition metal carbonate or carboxylate: 1,2-dicyanobenzene: 2-aminopyridine.

Optionally, there can be used as a promoter in the process of the invention an alkaline earth metal carbonate wherein the alkaline earth metal is magnesium, calcium, strontium or barium or any mixture thereof. The alkaline earth metal carbonate can be present in an amount of from 0-20% based on the transition metal carbonate or carboxylate.

Reaction time will vary inversely with temperature and will depend on the transition metal carbonate being used as a reactant. Reaction times of from a few minutes to about five hours are common.

Starting materials for the process of the invention are commercially available. The 2-aminopyridine can be a single compound or can be a mixture of isomers,

such as a mixture of 2-amino-3-, 4-, and 5-methylpyridines.

The process of the invention offers a one-step synthesis which does not use preformed ligand or solvent and gives a halide-free product. The compositions produced by the process of the invention are useful as catalyst in the decomposition of cyclohexyl hydroperoxide as described in part in copending application Serial No. 184,635 (Attorney's Docket No. CR 7856 A), filed simultaneously herewith.

Another aspect of the present invention is an improved process for producing 1,3-bis(2-pyridylimino)-isoindolines. The 1,3-bis(2-pyridylimino)isoindoline compounds prepared by the improved process of the invention have the formula

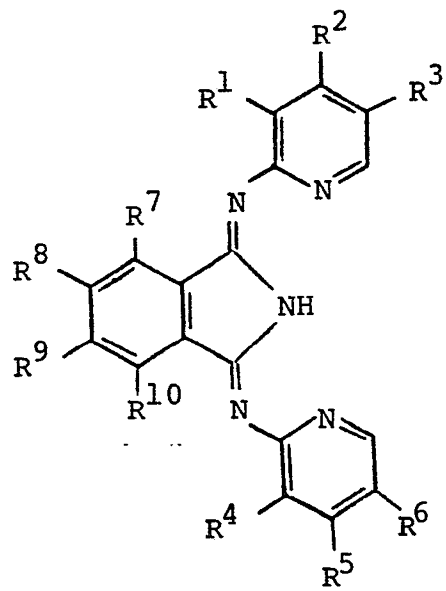

wherein

$R^1$, $R^2$, $R^3$, $R^4$, $R^5$ and $R^6$ are independently hydrogen, lower alkyl, lower alkoxyalkyl, phenyl, benzyl or phenethyl or any two adjacent members of $R^1$ through $R^6$ are jointly four CH entities of a benzene ring fused to the pyridine ring;

$R^7$ through $R^{10}$ are independently hydrogen, lower alkyl or lower alkoxyalkyl or any two adjacent members of $R^7$ through $R^{10}$ can jointly be four CH entities of a benzene ring fused to the benzene ring of the isoindoline moiety.

$R^1$ through $R^{10}$ have the preferred values previously given for the transition metal complexes. Isoindoline compounds produced by the process of the invention include 1,3-bis(2-pyridylimino)isoindoline [HBPI]; 1,3-bis(3-methyl-2-pyridylimino)isoindoline, isomers and mixtures thereof; 1,3-bis(3,4-dimethyl-2-pyridylimino)isoindoline; 1,3-bis(3-ethyl-2-pyridylimino)-isoindoline; 1,3-bis(3-butyl-2-pyridylimino)isoindoline; 1,3-bis(4-pentyl-2-pyridylimino)isoindoline; 1,3-bis(4-isohexyl-2-pyridylimino)isoindoline; 1,3-bis(5-phenyl-2-pyridylimino)isoindoline, 5-t-butyl-1,3-bis(2-pyridylimino)isoindoline; 4,7-dimethyl-1,3-bis(3-methyl-2-pyridylimino)isoindoline; 4,5,6-trimethyl-1,3-bis(2-pyridylimino(isoindoline; 4,5,6,7-tetrakis(t-pentyl)-1,3-bis(2-pyridylimino)isoindoline; 4-hexyl-1,3-bis(2-pyridylimino)isoindoline, and 1,3-bis(1-isoquinolylimino)isoindoline.

In the process of the invention the 1,3-bis(2-pyridylimino)isoindoline compound is prepared by contacting in the molten phase a 1,2-dicyanobenzene of the formula

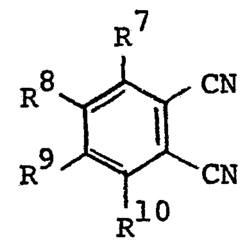

where $R^7$ through $R^{10}$ are as previously defined, with at least one 2-aminopyridine of the formula

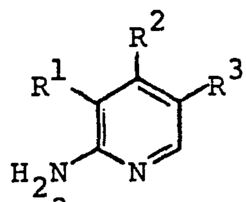

,

wherein $R^1$ through $R^3$ are as previously defined, optionally in the presence of an alkaline earth metal carbonate, at a temperature of from about 150°-300°C.

Preferably the temperature is from about 170°-220°C. Reactant ratios are not critical and will usually be 1:2 for the molar ratio 1,2-dicyanobenzene to 2-aminopyridine. A slight excess of the 2-aminopyridine can help increase the yield; however, use of excess 2-aminopyridine must be balanced against the need to recover unreacted material.

The amount of alkaline earth metal carbonate used in the present process is from about 0-20 mole % based on the 1,2-dicyanobenzene reactant. As with the production of the transition metal complex, the alkaline earth metal of the carbonate can be magnesium, calcium, strontium or barium or any mixture thereof.

Reaction time will vary inversely with temperature and with the percent of molar excess, if any, of the 2-aminopyridine compound. Usually, the reaction time will be from about 2-10 hours.

The present process for preparing a 1,3-bis(2-pyridylimino)isoindoline compound provides a solvent-free, halide-free, synthesis offering reduced reaction times as compared to prior art processes. The compounds produced can be converted by known methods to transition metal chelates which are useful as catalysts for the decomposition of cyclohexyl hydroperoxide.

The invention is further illustrated by the following examples in which temperatures are in degrees Centigrade and percentages are by weight unless otherwise stated.

EXAMPLE 1

Preparation of 1,3-bis(3-methyl-2-pyridylimino)isoindoline [H3MeBPI]

A mixture of 64.07 g (0.5 mole) of 1,2-dicyanobenzene, 123.9 g (1.15 mole) of 2-amino-3-methylpyridine, and 6.5 g (0.065 mole) of calcium carbonate was heated with stirring under nitrogen at about 180° for seven hours, after which the resulting melt was poured into 1.5 l of methanol, thereby causing the precipitation of a solid. The solid was separated by filtration, washed with methanol, and dried under reduced pressure to give 96.7 g (59%) of H3MeBPI. The product was shown to be pure by its proton NMR spectrum in deutorochloroform.

EXAMPLE 2

Preparation of H3MeBPI

A glass pressure vessel equipped with a stirrer and a pressure gauge was charged with 12.8 g (0.10 mole) of 1,2-dicyanobenzene, 23.6 g (0.22 mole) of 2-amino-3-methylpyridine, and 1.5 g (0.015 mole) of calcium carbonate. The vessel was sealed and placed in an oil bath at 210°. The resulting mixture was stirred for 3 hours and 20 minutes at an oil-bath temperature of about 192°-210°. During this time the pressure in the vessel gradually rose from 0 psig (0 kPa) to 34 psig (234 kPa). The pressure rise corresponded to the formation of by-product ammonia. The mixture was cooled to room temperature and allowed to stand for two days. It was then digested with about 50 ml of methanol and was filtered to obtain a solid which was washed with about 100 ml of methanol and dried under reduced pressure to give 13.4 g (41%) of H3MeBPI.

EXAMPLE 3

Preparation of H3MeBPI

A mixture of 12.8 g (0.10 mole) of 1,2-dicyanobenzene, 23.4 g (0.22 mole) of 2-amino-3-methylpyridine, and 1.5 g (0.015 mole) of calcium carbonate was reacted by a procedure similar to that described in Example 2, except that the reaction mixture was heated in the oil bath for five hours at an oil-bath temperature of 196-205°C and the pressure gradually rose from 0 psig to 48 psig (331 kPa). The yield of H3MeBPI was 19.7 g (60%).

EXAMPLE 4

Preparation of H3MeBPI

A glass reactor open to the atmosphere was charged with 12.8 g (0.10 mole) of 1,2-dicyanobenzene, 32.4 g (0.30 mole) of 2-amino-3-methylpyridine, and 1.5 g (0.015 mole) of calcium carbonate to provide a mixture which was then blanketed with nitrogen. The reactor was placed in an oil bath at 188° and stirring was started. The oil-bath temperature was raised to 195° over a 15-minute period. The mixture was heated for 4.75 hr under nitrogen with stirring at an oil-bath temperature of 194-195° and a reaction temperature of 178-182°. The resulting product was cooled to room temperature and worked up as in a manner similar to that described in Example 2. The yield of H3MeBPI was 26.1 g (80%).

The product was extracted with methylene chloride, and the extracts were evaporated to dryness. The residual solid was 22.1 g (68%) of H3MeBPI, which was shown to be pure by its proton NMR spectrum.

EXAMPLE 5

Preparation of H3MeBPI and Conversion to Cobalt(II) Derivative of 1,3-Bis(3-methyl-2-pyridylimino)isoindoline [Co(3MeBPI)$_2$]

A glass flask equipped with mechanical stirrer, and condenser, was charged with 1,2-dicyanobenzene (30 g, 0.23 mole) and 2-amino-3-methylpyridine (50.64 g, 0.468 mole). The flask was placed in an oil bath which was then heated to 210° for 3 hr (reaction temperature: 194-196°). Measurement of ammonia evolution indicated that conversion to H3MeBPI ceased after 3 hr. At this point, NMR indicated greater than 80% conversion to H3MeBPI.

This product was used to make a transition metal complex as described below. After cooling to ambient temperature, 75 ml of toluene was added to the product to improve stirring. The temperature was then increased to 80° and sodium hydroxide (9.36, 0.23 mole) in methanol (45 ml) was added. To the resulting slurry was then added, in one portion, cobalt acetate (28.57, 0.12 mole) in 300 ml of methanol. The resulting brick red slurry was heated for 1 hr. The mixture was then cooled to 40° and Co(3MeBPI)$_2$ was separated by filtration through a coarse porosity fritten disc funnel and washed with 300 ml of methanol followed by 150 ml of diethyl ether to facilitate drying. The yield of Co(3MeBPI)$_2$ was 81.6 g (96%).

EXAMPLE 6

Preparation of Mixed Methyl HBPI and Conversion to Co(Mixed MeBPI)$_2$

A glass flask equipped with condenser, magnetic stir bar, thermometer and nitrogen inlet was charged with 1,2-dicyanobenzene (32 g, 0.25 mole), aminated mixed methylpyridines (54.07, 0.5 mole) [55% 2-amino-3-methylpyridine and 45% 2-amino-4-methylpyridine, by

proton NMR] and calcium carbonate (5 g, 0.05 mole). The flask containing the resulting mixture was placed in a 205° oil bath and heated for 5 hr, 15 min to obtain a melt.

The resulting melt was cooled to ~150°, then poured into 1.2 l of methanol to obtain a green solution to which was added sodium hydroxide (9.05, 0.23 mole) followed by cobalt acetate (27.97 g, 0.112 mole). After stirring at ambient temperature the methanol was removed in vacuo to yield a crystalline brick-red product which was a mixture of different isomeric $Co(MeBPI)_2$ species.

EXAMPLE 7

Preparation of $Co(3MeBPI)_2$

A mixture of 12.8 g (0.10 mole) of 1,2-dicyanobenzene, 23.6 g (0.21 mole) of 2-amino-3-methylpyridine, and 6.0 g (0.05 mole) of cobalt carbonate was heated under nitrogen in an oil bath at 125-135° for 1 hour and 23 minutes, after which it was poured while still hot into about 400 ml of methanol thereby causing formation of a solid. The resulting mixture was stirred for about two minutes and filtered to obtain the solid which was dried under reduced pressure to give 5.8 g (16%) of $Co(3MeBPI)_2$, identified by its proton NMR spectrum.

EXAMPLE 8

Preparation of $Co(3MeBPI)_2$

This example was performed using a procedure substantially similar to that described in Example 7, except that twice as much of each reactant was used and the resulting mixture was heated in an oil bath at about 170° for 5/3 hours and was cooled to room temperature before being combined with methanol. The yield of Co(3MeBPI), again identified by its proton NMR spectrum, was 25.0 g (37%).

## EXAMPLE 9

### Preparation of $Co(3MeBPI)_2$

A glass reactor equipped with a stirrer and thermometer was charged with 12.8 g (0.10 mole) of 1,2-dicyanobenzene, 23.6 g (0.21 mole) of 2-amino-3-methylpyridine, 5.9 g (0.05 mole) of cobalt carbonate, and 1.5 g (0.015 mole) of calcium carbonate. The resulting mixture was placed in an oil bath at 112° and heated with stirring under nitrogen. The oil-bath temperature was raised to 182°C over 35 minutes and was then held at 180-194° for 50 minutes, thereby maintaining a reaction mixture temperature of 143-172°. The mixture was solidified by cooling. The resulting solid product was washed with methanol and digested at room temperature with methylene chloride to provide a mixture which was filtered to give a filtrate which was evaporated to dryness, yielding 5.4 g (15%) of $Co(3MeBPI)_2$.

## EXAMPLES 10-13

These examples show the effect which a change in the reaction time has on the yield. A procedure substantially similar to that described in Example 9 was used with the exceptions noted in the following table where the results are also summarized. The data for Example 9 are included for comparison. For Examples 11-13 the solid product was washed with petroleum ether instead of being extracted with methylene chloride.

TABLE

| Example | Bath Temp °C | Reaction Mixture Temp °C | Time Hr:Min | Grams of Product | % Yield |
|---|---|---|---|---|---|
| 9 | 180-194 | 143-172 | 0:50 | 5.4 | 15 |
| 10 | 170-186 | 137-200 | 1:35 | 13.7 | 38 |
| 11 | 181-190 | 154-185 | 2:43 | 20.4 | 57 |
| 12 | 188-191 | 173-186 | 3:43 | 23.0 | 65 |
| 13 | 181-187 | 175-180 | 4:43 | 21.0 | 59 |

In Examples 14-16 all reactions were conducted in a 250 ml flask equipped with a thermometer, stirrer and a nitrogen inlet. Reaction mixtures were heated with a controlled oil bath. In these examples the expression $M(3MeBPI)_2$ represents the transition metal complex of 1,3-bis(3-methyl-2-pyridylimino)isoindoline.

EXAMPLE 14

Preparation of $Cu(3MeBPI)_2$

The reaction vessel was charged with 12.8 g (0.10 mole) of 1,2-dicyanobenzene, 23.6 g (0.21 mole) of 2-amino-3-methylpyridine, 6.12 g (0.05 mole) of basic copper carbonate, $Cu_2(CO_3)(OH)_2$, and 1.5 g (0.015 mole) of calcium carbonate to provide a reaction mixture which was heated under nitrogen at an oil-bath temperature of 135° for 24 minutes. The resulting product was washed with 200 ml of methanol, then with 200 ml of petroleum ether and finally with 800 ml of dichloromethane and then dried to give 17.87 g (49.9%) of crystalline $Cu(3MeBPI)_2$.

EXAMPLE 15

Preparation of $Zn(3MeBPI)_2$

The ingredients added to the reaction vessel were substantially similar to those of Example 14, however 6.21 g (0.05 mole) of $ZnCO_3$ was used instead of basic copper carbonate. The reaction mixture was

heated for 32 minutes at an oil-bath temperature of 160° to yield a brilliant green product which was washed with 200 ml of methanol and 500 ml of dichloromethane. The washed product was extracted with 500 ml of hot chloroform to provide an extract solution which was filtered. The resulting filtrate was evaporated to dryness to give 11.5 g (32%) of brilliant yellow Zn(3MeBPI)$_2$.

EXAMPLE 16

Preparation of Ni(3MeBPI)$_2$

The ingredients added to the reaction vessel were substantially similar to those used in Example 14 except that 6.00 g (0.05 mole) of $NiCO_3$ was employed instead of basic copper carbonate. The reaction mixture was heated at an oil-bath temperature of 150° to yield a red-brown product which was washed with 300 ml of methanol and 750 ml of dichloromethane to give 3.05 g (8.5%) of orange, crystalline Ni(3MeBPI)$_2$.

EXAMPLE 17

In situ Preparation of Co(3MeBPI)$_2$

A 500 ml glass pressure vessel equipped with a magnetic stirrer and a pressure gauge was charged with 1,2-dicyanobenzene (12.8 g, 0.10 mole), 2-amino-3-methylpyridine (21.6 g, 0.20 mole), calcium carbonate (1.5 g, 0.015 mole), and cobalt octoate (cobalt bis(2-ethyl hexanoate)) (17.26 g, 0.05 mole). The vessel was sealed and placed in an oil bath at 153°. The resulting mixture quickly became thick and after 20 minutes, turned into a solid reaction mass. After cooling to ambient temperature, a small portion of the resulting crude reaction mixture was tested as a catalyst for cyclohexylhydroperoxide decomposition and found to be active.

To assess the yield of Co(3MeBPI)$_2$ in the catalyst mixture, work up was continued. The reaction

mass was suspended in methanol (300 ml) and filtered to obtain a solid which was dried under vacuum yielding 27.9 g (79%) of $Co(3MeBPI)_2$ that was identified by NMR.

EXAMPLE 18

In Situ Preparation of $Co(BPI)_2$

A glass flask equipped with magnetic stirring bar was charged with 1,2-dicyanobenzene (12.8 g, 0.10 mole), 2-aminopyridine (18.8 g, 0.20 mole), calcium carbonate (1.5 g, 0.015 mole) and cobalt octoate (17.3 g, 0.05 mole). The mixture was heated in an oil bath to 150-155°. The resulting purple slurry rapidly turned brown and in 17 minutes had become a gelatinous brown reaction mass, after which heating was discontinued. This product was an active catalyst for the decomposition of cyclohexylhydroperoxide.

EXAMPLE 19

In Situ Preparation of $Fe(3MeBPI)_2$

A glass flask equipped with stirring bar, thermometer and bubbler was charged with 1,2-dicyanobenzene (12.8 g, 0.1 moles), 2-amino-3-methylpyridine (21.6 g, 0.2 moles), and ferrous naphthenate (19.9 g, 0.05 moles). The flask was placed in an oil bath which was heated to 165°C. The reaction was slightly exothermic, the temperature of the contents of the flask rising to 175°C. Measurement of ammonia evolution indicated that the reaction was complete after 50 minutes. The resulting mixture was cooled to room temperature to obtain 51.76 g of composition which contained 29.1 g (82%) $Fe(3MeBPI)_2$ as measured after filtration, washing with petroleum ether, and drying.

EXAMPLE 20

In Situ Preparation of $Co(3MeBPI)_2/Fe(3MeBPI)_2$

The procedure used in this example was substantially similar to that of Example 19 except that

ferrous naphthenate (6.56 g) and cobalt octoate (11.59 g) were used. The oil bath was at 160° and the reaction exothermed to 173°. Gas evolution ceased after 30 minutes. Crystalline product was observed in the viscous reaction mixture. The yield of reaction mixture was 44.9 g which contained 26.1 g (75%) of dark red brown crystals that were a mixture of $Co(3MeBPI)_2$ and $Fe(3MeBPI)_2$.

EXAMPLE 21

In Situ Preparation of Co(3MeBPI)(Acetate)

A glass flask was charged with 2-amino-3-methylpyridine (54.0 g, 0.50 mole), 1,2-dicyanobenzene (32.0 g, 0.25 mole) and cobalt acetate (31 g, 0.125 mole). The flask was placed in a 160° oil bath and the contents of the flask melted. Within 5 minutes, the contents of the flask had become a brick red solid mass indicating extensive conversion to Co(3MeBPI)(acetate).

CLAIMS

1. A process for preparing a composition consisting essentially of at least one transition metal complex having the formula

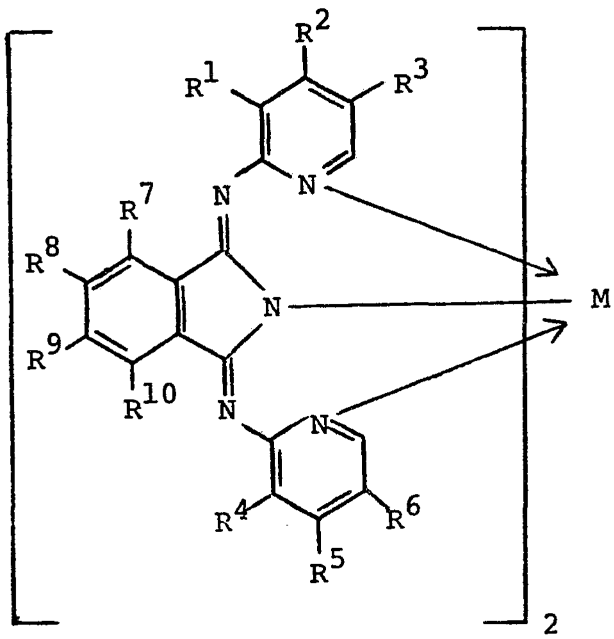

I

or

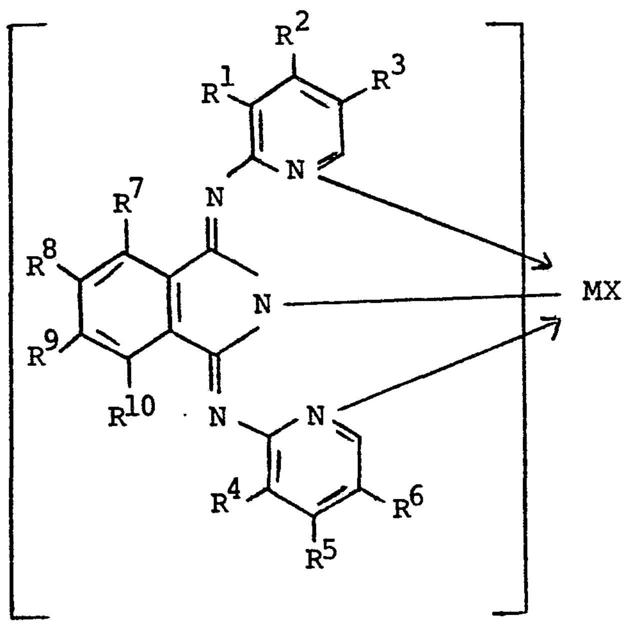

II

wherein

M is a divalent transition metal selected from Co, Mn, Fe, Ni, Pd, Zn, Cu or Ru;

X is a carboxylate group;

$R^1$, $R^2$, $R^3$, $R^4$, $R^5$, and $R^6$ are independently hydrogen, lower alkyl, lower alkoxyalkyl, phenyl, benzyl, or phenethyl or any two adjacent members of $R^1$ through $R^6$ are jointly four CH entities of a benzene ring fused to the pyridine ring;

$R^7$, $R^8$, $R^9$ and $R^{10}$ are independently hydrogen, lower alkyl or lower alkoxyalkyl or any two adjacent members of $R^7$ through $R^{10}$ can jointly be four CH entities of a benzene ring fused to the benzene ring of the isoindoline moiety;

with the proviso that the values of $R^1$ through $R^{10}$ can be different for each ligand;

comprising contacting in a molten phase a 1,2-dicyanobenzene of the formula

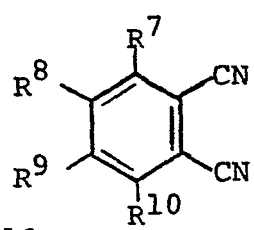

,

where $R^7$ through $R^{10}$ are as previously defined, with at least one 2-aminopyridine of the formula

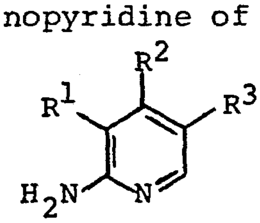

,

wherein $R^1$ through $R^3$ are as previously defined, in the presence of a transition metal, M, carbonate or a carboxylate of the formula $MX_2$ where M and X are as previously defined, and optionally in the presence of

an alkaline earth metal carbonate, at a temperature of from about 100°-300°C.

2. The process of Claim 1 where M is Co, Mn or Fe or a combination thereof and the transition metal complex is of formula I.

3. The process of claim 2 wherein the temperature is from about 150° to 220°C.

4. The process of Claim 3 wherein the mole ratio of transition metal carbonate or carboxylate to 1,2-dicyanobenzene to 2-aminopyridine is about 1:1:2.

5. The process of Claim 3 wherein $R^7$ through $R^{10}$ are independently lower alkyl or hydrogen.

6. The process of Claim 5 wherein $R^1$ through $R^6$ are independently hydrogen, methyl or t-butyl.

7. The process of Claim 6 wherein any two of $R^1$, $R^2$ and $R^3$ and any two of $R^4$, $R^5$ and $R^6$ are hydrogen, and $R^7$ through $R^{10}$ are hydrogen

8. The process of Claim 7 wherein $R^1$ and $R^4$ are methyl.

9. The process of Claim 7 wherein $R^2$ and $R^5$ are t-butyl.

10. The process of Claim 6 wherein $R^1$ through $R^{10}$ are hydrogen.

11. A process for preparing a compound of the formula

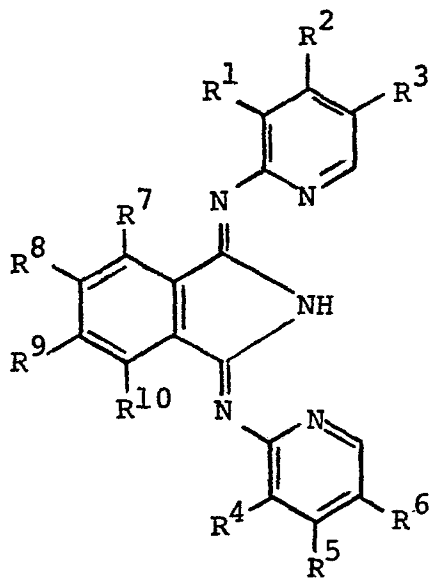

wherein

$R^1$, $R^2$, $R^3$, $R^4$, $R^5$ and $R^6$ are independently hydrogen, lower alkyl, lower alkoxyalkyl, phenyl, benzyl or phenethyl or any two adjacent members of $R^1$ through $R^6$ are jointly four CH entities of a benzene ring fused to the pyridine ring; and

$R^7$ through $R^{10}$ are independently hydrogen, lower alkyl or lower alkoxyalkyl or any two adjacent members of $R^7$ through $R^{10}$ can jointly be four CH entities of a benzene ring fused to the benzene ring of the isoindoline moiety;

comprising contacting in a molten phase a 1,2-dicyanobenzene of the formula

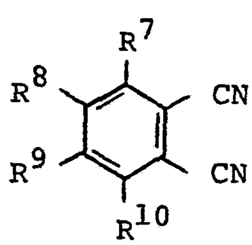

,

where $R^7$ through $R^{10}$ are as previously defined, with at least one 2-aminopyridine of the formula

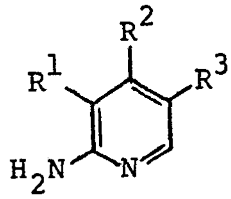

,

wherein $R^1$ through $R^3$ are as previously defined, optionally in the presence of an alkaline earth metal carbonate at a temperature of from about 150°-300°C.

12. The process of Claim 11 wherein the temperature is from about 170°-220°C.

13. The process of Claim 12 wherein the alkaline earth metal carbonate is present in a mole percentage of from about 0-20% based on the 1,2-dicyanobenzene.

14. The process of Claim 12 wherein 1,2-dicyanobenzene and the 2-aminopyridine are present in a mole ratio of about 1:2 respectively.

15. The process of Claim 12 wherein $R^7$ through $R^{10}$ are independently hydrogen or lower alkyl.

16. The process of Claim 15 wherein $R^1$ through $R^3$ are independently hydrogen, methyl or t-butyl.

17. The process of Claim 16 wherein any two of $R^1$, $R^2$ and $R^3$ are hydrogen and $R^7$ through $R^{10}$ are hydrogen.

18. The process of Claim 17 wherein $R^1$ is methyl.

19. The process of Claim 17 wherein $R^2$ is t-butyl.

20. The process of Claim 16 wherein $R^1$, $R^2$ and $R^3$ and $R^7$, $R^8$, $R^9$ and $R^{10}$ are hydrogen.

European Patent Office

# EUROPEAN SEARCH REPORT

0048024

Application number

EP 81 10 7301

## DOCUMENTS CONSIDERED TO BE RELEVANT

| Category | Citation of document with indication, where appropriate, of relevant passages | Relevant to claim |
|---|---|---|
| D | JOURNAL OF ORGANIC CHEMISTRY, vol. 42, no. 6, 18th March 1977, pages 1872-1878<br>U.S.A.<br>W.O. SIEGL: "Metal ion activation of nitriles. Syntheses of 1,3-bis(arylimino)isoindolines"<br>* Complete disclosure *<br>-- | 1-20 |
| | CHEMICAL ABSTRACTS, vol. 88, no. 20, 15th May 1978, page 620, no. 145368c<br>Columbus, Ohio, U.S.A.<br>W.O. SIEGL: "A new bis-chelating ligand system. Synthesis and chelating behavior"<br>& INORG. CHIM. ACTA 1977, 25(2), L65-L66<br>* Abstract *<br>---- | 1-20 |

CLASSIFICATION OF THE APPLICATION (Int. Cl.3)

C 07 D 401/12

TECHNICAL FIELDS SEARCHED (Int. Cl.3)

C 07 D 401/12
C 09 B 57/04
57/00
26/02

CATEGORY OF CITED DOCUMENTS

- X: particularly relevant
- A: technological background
- O: non-written disclosure
- P: intermediate document
- T: theory or principle underlying the invention
- E: conflicting application
- D. document cited in the application
- L: citation for other reasons
- &: member of the same patent family, corresponding document

The present search report has been drawn up for all claims

| Place of search | Date of completion of the search | Examiner |
|---|---|---|
| The Hague | 26-10-1981 | MAISONNEUVE |